# EUROPÄISCHE PATENTANMELDUNG

(11) **EP 0 639 573 A1**
(43) Veröffentlichungstag der Anmeldung: **22.02.1995**
(21) Anmeldenummer: 94111765.7
(22) Anmeldetag: 28.07.1994
(51) Int. Cl.: C07D 307/85, C07D 277/68, C07D 333/70, C07D 249/18, C07D 235/32, C07D 209/42, A61K 31/33

(54) **Benzokondensierte 5-Ringheterocyclen, Verfahren zu ihrer Herstellung, ihre Verwendung als Medikament, ihre Verwendung als Diagnostikum, sowie sie enthaltendes Medikament**

(30) Priorität: 03.08.1993 DE 4326005; 25.04.1994 DE 4414316
(71) Anmelder: HOECHST AKTIENGESELLSCHAFT, D-65929 Frankfurt am Main (DE)
(72) Erfinder: Lang, Hans-Jochen, Dr., D-65719 Hofheim (DE); Weichert, Andreas, Dr., D-663329 Egelsbach (DE); Schwark, Jan-Robert, Dr., D-65929 Frankfurt (DE); Scholz, Wolfgang, Dr. Unterortstrasse 30, D-65929 Frankfurt (DE); Albus, Udo, Dr., D-61197 Florstadt (DE); Crause, Peter, Dr., D-63069 Offenbach (DE)

(57) **Zusammenfassung**

Beschrieben werden benzokondensierte 5-Ringheterocyclen der Formel I 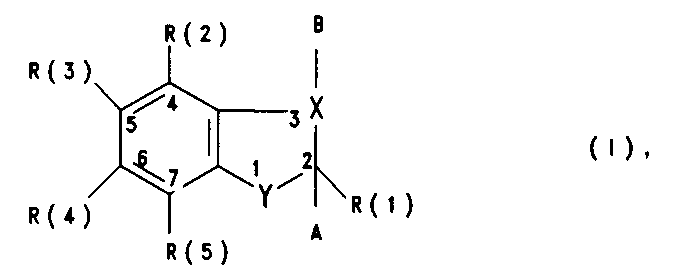
mit X gleich N oder CR(6); Y gleich Sauerstoff, S oder NR(7); A und B gemeinsam eine Bindung oder beide Wasserstoff, sofern gleichzeitig X gleich CR(6) und Y gleich NR(7) sind,
einer der Substituenten R(1) bis R(6) eine -CO-N = C(NH₂)₂-Gruppe; die jeweils anderen Substituenten R(1) bis R(6) H, Hal, Alkyl;
bis zu zwei der anderen Substituenten R(1) bis R(6) CN, NO₂, N₃, (C₁-C₄)-Alkyloxy, CF₃; bis zu einem der anderen Substituenten R(8)-CₙH₂ₙ-Z-, Phenyl; R(7) gleich H, Alk(en)yl, R(8)-CₙH₂ₙ-, sowie deren pharmazeutisch verträgliche Salze.
Beschrieben wird ferner ein Verfahren zur Herstellung der Verbindungen I, welches darin besteht, daß man eine Verbindung der Formel II 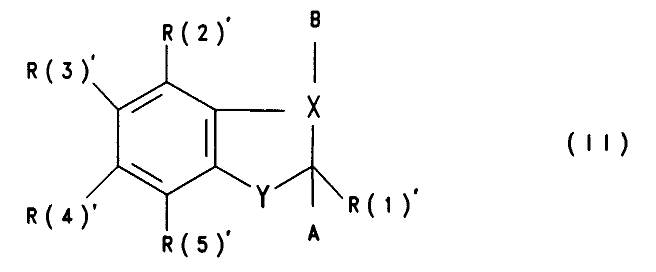
worin einer der Substituenten R(1)' bis R(5)' eine -CO-L -Gruppe bedeutet und L für eine leicht nucleophil substituierbare leaving group steht, mit Guanidin umsetzt, und daß man gegebenenfalls in das pharmakologisch verträgliche Salz überführt.

## Beschreibung

Die Erfindung betrifft benzokondensierte 5-Ringheterocyclen der Formel I 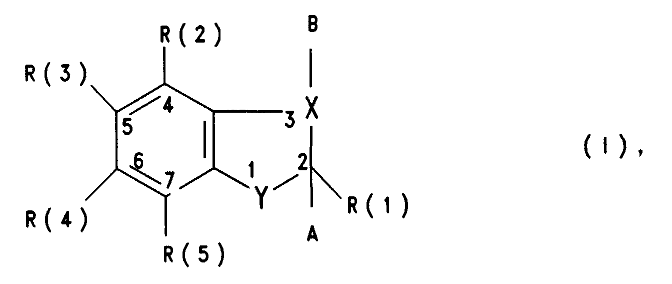
worin bedeuten:
- X: N, CR(6),
- Y: Sauerstoff, S, NR(7),
- A, B: gemeinsam eine Bindung
oder
beide Wasserstoff, sofern gleichzeitig X gleich CR(6) und Y gleich NR(7) sind,

einer der Substituenten R(1) bis R(6) eine -CO-N = C(NH₂)₂-Gruppe, die jeweils anderen Substituenten R(1) bis R(6)
Wasserstoff, F, Cl, Br, I, (C₁-C₆)-Alkyl,
bis zu zwei der anderen Substituenten R(1) bis R(6)
CN, NO₂, N₃, (C₁-C₄)-Alkyloxy, CF_{3,}
bis zu einem der anderen Substituenten
R(8)-CₙH₂ₙ-Z-,
- n: Null bis 10,
wobei die Alkylenkette -CₙH₂ₙ- geradkettig oder verzweigt ist und wobei ein C-Atom durch ein Sauerstoff- oder S-Atom oder durch ein N-Atom ersetzt sein kann,
- R(8): Wasserstoff, (C₂-C₆)-Alkenyl, (C₃-C₁₀)-Cycloalkyl,
das unsubstituiert oder durch 1 bis 4 Methylgruppen oder eine OH-Gruppe substituiert ist, oder eine Ethylengruppe -CH = CH- enthalten kann, und worin eine Methylengruppe durch ein Sauerstoff- oder S-Atom oder durch ein N-Atom ersetzt sein kann,
Phenyl,
unsubstituiert oder substituiert durch 1 bis 3 Substituenten ausgewählt aus der Gruppe bestehend aus F, Cl, Br, I, CF₃, CH₃-S(O)ₛ-,
- s: Null, 1, 2,
R(9)-W_{y}-,
- R(9): H, Methyl, Ethyl,
- W: gleich Sauerstoff, NR(10),
- R(10): H, Methyl,
- y: Null, 1,
CₘF₂ₘ₊₁,
- m: 1 bis 3,
1- oder 2-Naphthyl, Pyridyl, Chinolyl, Isochinolyl,
- Z: -CO-, -CH₂-, -[CR(11)(OH)]_{q}-
- q: 1, 2, 3,
- R(11): H, Methyl,
Sauerstoff, -NR(12)-,
- R(12): H, Methyl,
-S(O)ₛ-,
- s: Null, 1, 2,
-SO₂-NR(13)-,
- R(13): H, (C₁-C₄)-Alkyl,
- R(7): Wasserstoff, (C₁-C₁₀)-Alkyl, (C₂-C₁₀)-Alkenyl, R(8)-CₙH₂ₙ-,

sowie deren pharmazeutisch verträgliche Salze.

Enthalten die Substituenten R(1) bis R(5) ein oder mehrere Asymmetriezentren, so gehören sowohl S wie auch R konfigurierte Verbindungen zur Erfindung. Die Verbindungen können als optische Isomere, als Racemate oder als Gemische derselben vorliegen.

Die bezeichneten Alkylreste können sowohl geradkettig wie verzweigt vorliegen.

Bevorzugt sind Verbindungen der Formel I, worin bedeuten:
- X: CR(6), N,
- Y: NR(7),
- A, B: gemeinsam eine Bindung
oder
beide Wasserstoff, sofern gleichzeitig X gleich CR(6) und Y gleich NR(7) sind,
und die Reste R(1) bis R(7) die angegebene Bedeutung besitzen.

Besonders bevorzugt sind Verbindungen der Formel I, worin bedeuten:
- X: CR(6), N,
- Y: NR(7),
- A, B: gemeinsam eine Bindung
oder
beide Wasserstoff, sofern gleichzeitig X gleich CR(6) und Y gleich NR(7) sind,
einer der Substituenten R(1) bis R(6) eine -CO-N = C(NH₂)₂-Gruppe, und die jeweils anderen Substituenten R(1) bis R(6)
Wasserstoff, F, Cl, Br, I, (C₁-C₆)-Alkyl,
bis zu zwei der anderen Substituenten R(1) bis R(6)
CF₃, (C₁-C₄)-Alkyloxy,
bis zu einem der anderen Substituenten R(1) bis R(6)
CN, NO₂, N₃, R(8)-CₙH₂ₙ-Z-,
- n: Null bis 4, wobei die Alkylenkette -CₙH₂ₙ- geradkettig oder verzweigt sein kann und ein C-Atom durch ein Sauerstoff- oder S-Atom oder durch ein N-Atom ersetzt sein kann,
- R(8): Wasserstoff, (C₃-C₆)-Alkenyl, (C₅-C₈)-Cycloalkyl,
das unsubstituiert ist oder durch 1 - 2 Methylgruppen oder eine OH-Gruppe substituiert, und worin eine Methylengruppe durch ein Sauerstoff- oder S-Atom oder durch ein N-Atom ersetzt sein kann,
Phenyl,
das unsubstituiert ist oder substituiert mit 1 bis 3 Substituenten ausgewählt aus der Gruppe bestehend aus F, Cl, Br, I, CF₃, CH₃-S(O)ₛ-
- s: Null, 1, 2,
R(9)-W_{y}-
- R(9): H, Methyl, Ethyl,
- W: Sauerstoff, NR(10),
- R(10): H, Methyl,
- y: Null, 1,
CₘF₂ₘ₊₁,
- m: 1 bis 3,
Pyridyl, Chinolyl, Isochinolyl
- Z: -CO-, -CH₂-, Sauerstoff, -NR(12)-,
- R(12): H, Methyl,
-S(O)ₛ-,
- s: Null, 1, 2,
-SO₂-NR(13)-,
- R(13): H, (C₁-C₄)-Alkyl,
- R(7): Wasserstoff, (C₁-C₆)-Alkyl, (C₂-C₄)-Alkenyl, R(8)-CₙH₂ₙ-.

Insbesondere bevorzugt sind Verbindungen der Formel I, worin bedeuten:
- X: CR(6),
- Y: NR(7),
- A, B: gemeinsam eine Bindung
oder
beide Wasserstoff, sofern gleichzeitig X gleich CR(6) und Y gleich NR(7)
sind,
und
R(1) -CO-N=C(NH₂)₂
und die jeweils anderen Substituenten R(2) bis R(6)
Wasserstoff, F, Cl, Br,
bis zu zwei der Substituenten R(2) bis R(6)
CF₃, (C₁-C₂)-Alkyloxy,
bis zu einem der Substituenten R(2) bis R(6)
R(8)-CₙH₂ₙ-Z-,
- n: Null, 1, 2,
wobei die Alkylenkette -CₙH₂ₙ- geradkettig oder verzweigt ist und wobei ein C-Atom durch ein Sauerstoff- oder S-Atom oder durch ein N-Atom ersetzt sein kann,
- R(8): Wasserstoff, Phenyl,
das unsubstituiert ist oder substituiert mit 1 bis 3 Substituenten ausgewählt aus der Gruppe bestehend aus F, Cl, CF₃, CH₃-S(O)ₛ-,
- s: Null, 2,
R(9)-W_{y}-,
- R(9): H, Methyl,
- W: Sauerstoff,
- y: Null, 1,
Pyridyl, Chinolyl, Isochinolyl,
- Z: -CO-, -CH₂-, -S(O)ₛ-, Sauerstoff,
- s: Null, 1, 2,
und
- R(7): (C₁-C₆)-Alkyl, (C₃-C₄)-Alkenyl, R(8)-CₙH₂ₙ-.

Ganz besonders bevorzugt sind die Verbindungen
5-Chlor-1-methyl-2-indolylcarbonyl-guanidin-hydrochlorid,
5-Chlor-1-ethyl-2-indolylcarbonyl-guanidin-hydrochlorid,
3-Chlor-1-methyl-2-indolylcarbonyl-guanidin-hydrochlorid,
3,5-Dichlor-1-methyl-2-indolylcarbonyl-guanidin-hydrochlorid,
5-Fluor-1-methyl-2-indolylcarbonyl-guanidin-hydrochlorid,
3-Chlor-5-fluor-1-methyl-2-indolylcarbonyl-guanidin-hydrochlorid,
4,6-Dichlor-1,3-dimethyl-2-indolylcarbonyl-guanidin-hydrochlorid,
2-Phenoxy-1-phenylindol-3-carbonsäureguanidid Methansulfonsäuresalz,
2-Chlor-1-phenylindol-3-carbonsäureguanidid Methansulfonsäuresalz,
1-Methylindolin-2-carbonsäure-guanidid-hydrochlorid,
5-Fluor-1-methylindolin-2-carbonsäure-guanidid-hydrochlorid.

Die Verbindungen I sind substituierte Acylguanidine.
Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 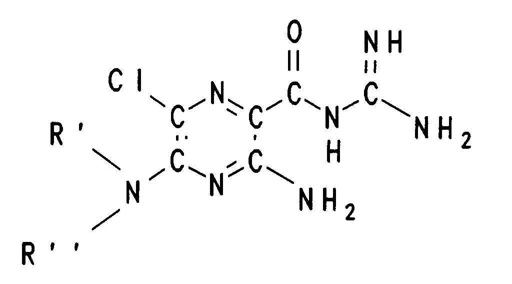
Amilorid: R', R'' = H
Dimethylamilorid: R', R'' = CH₃
Ethylisopropylamilorid: R' = C₂H₅, R'' = CH(CH₃)₂
Darüberhinaus sind Untersuchungen bekannt geworden, die auf antiarrhythmische Eigenschaften von Amilorid hinweisen (Circulation 79; 1257 bis 1263 (1989)). Einer breiten Anwendung als Antiarrhythmikum steht jedoch entgegen, daß dieser Effekt nur schwach ausgeprägt ist und von einer blutdrucksendenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten (Eur. Heart J. 9 (suppl. 1): 167 (1988) (book of abstracts)). So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

In der Europäischen Offenlegungsschrift 416 499 werden Benzoylguanidine mit antiarrhythmischen Eigenschaften beschrieben.

In der US-Patentschrift 3 780 027 werden ebenfalls Acylguanidine beschrieben, die sich grundlegend von den hier beschriebenen erfindungsgemäßen Verbindungen I dadurch unterscheiden, daß es sich um trisubstituierte Benzoylguanidine handelt, die sich in ihrem Substitutionsmuster von im Handel befindlichen Diuretika, wie Bumetanid und Furosemid, ableiten und eine für die angestrebte salidiuretische Wirkung wichtige Aminogruppe in Position 2 bzw. 3 zur Carbonylguanidingruppe tragen. Entsprechend wird für diese Verbindungen eine starke salidiuretische Wirksamkeit berichtet.

Es war daher überraschend, daß die erfindungsgemäßen Verbindungen keine unerwünschten und nachteiligen salidiuretischen, jedoch sehr gute antiarrhythmische Eigenschaften aufweisen, weshalb sie zum Behandeln von Krankheiten, wie sie beispielsweise bei Sauerstoffmangelerscheinungen auftreten, geeignet sind. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüberhinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüber hinaus zeichnen sich die erfindungsgemäßen Verbindungen der Formel I durch starke inhibierende Wirkung auf die Proliferationen von Zellen, beispielsweise der Fibroblasten-Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Die Erfindung betrifft weiterhin ein Verfahren zur Herstellung der Verbindungen I, dadurch gekennzeichnet, daß man

### Verbindungen der Formel II

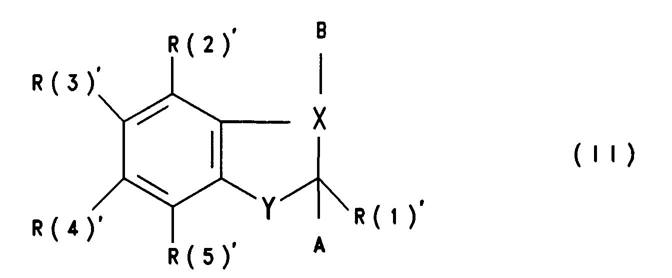

worin einer der Substituenten R(1)' bis R(5)' eine -CO-L -Gruppe bedeutet und L für eine leicht nucleophil substituierbare leaving group steht, und die jeweils anderen Substituenten R(1)' bis R(5)' die angegebene Bedeutung besitzen,
mit Guanidin unter Bildung der in Formel I angegebenen Acylguanidingruppe, -CO-N = C(NH₂)₂,
umsetzt, und daß man gegebenenfalls in das pharmakologisch verträgliche Salz überführt.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann. Neben den Carbonsäurechloriden der Formel II (L = Cl) lassen sich auch weitere aktivierte Säurederivate der Formel II in an sich bekannter Weise direkt aus den zugrundeliegenden heterocyclischen Carbonsäurederivaten (Formel II, L = OH) herstellen, wie beispielsweise die Methylester der Formel II mit L = OCH₃ durch Behandeln mit gasförmigem HCl in Methanol, die Imidazolide der Formel II durch Behandeln mit Carbonyldiimidazol (L = 1-Imidazolyl, Staab, Angew. Chem. Int. Ed. Engl. 1, 351 bis 367 (1962)), die gemischten Anhydride II mit Cl-COOC₂H₅ oder Tosylchlorid in Gegenwart von Triethylamin in einem inerten Lösungsmittel, wie auch die Aktivierungen von Carbonsäuren mit Dicyclohexylcarbodiimid (DCC). Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel I mit Guanidin erfolgt in an sich bekannter Weise in einem protischen oder aprotischen polaren aber inerten organischen Lösungsmittel. Dabei haben sich bei der Umsetzung der Carbonsäuremethylester (II, L = OMe) mit Guanidin Methanol oder THF zwischen 20°C und Siedetemperatur als Lösungsmittel bewährt. Bei den meisten Umsetzungen von Verbindungen II mit Guanidin wurde vorteilhaft in aprotischen inerten Lösungsmitteln wie THF, Dimethoxyethan, Dioxan gearbeitet. Aber auch Wasser kann als Lösungsmittel bei der Umsetzung von II und Guanidin verwendet werden.

Wenn L = Cl bedeutet, arbeitet man vorteilhaft unter Zusatz eines Säurefängers, z. B. in Form von überschüssigen Guanidin zur Abbindung der Halogenwasserstoffsäure.

Ein Teil der zugrundeliegenden heterocyclischen Carbonsäure-Derivate ist bekannt und in der Literatur beschrieben. Die unbekannten heterocyclischen Carbonsäuren II (L = OH) können nach literaturbekannten Methoden hergestellt werden.

So erhält man beispielsweise die 2-Benzimidazolcarbonsäuren [Formel II mit R(1) = -COOH, X = N und Y = -NR(7)], welche zu bevorzugten erfindungsgemäßen Verbindungen führen, in an sich bekannter Weise durch Oxidation der entsprechenden 2-Hydroxymethyl-benzimidazole mit Kaliumpermanganat analog Bistrzycki und Przeworski (Ber. 45, 3483 [1912]).

Die zu bevorzugten Carbonylguanidinen der Formel I führenden Indol-2-carbonsäuren erhält man beispielsweise aus den 2-Nitrotoluolen nach J. R. Johnson et al. J. Am. Chem. Soc. 67, 423 (1945) [siehe auch Noland und Baude, Org. Synth. Coll. Vol. 5, 567 (1973)], nach der Fischer-Indolsynthese aus Brenztraubensäure und Phenylhydrazon. Eine weitere Darstellungsmethode für Indol-2-carbonsäuren besteht in der Bromoform- oder Jodoform-Reaktion von 2-Acetylindolen, die nach Rajur et al., Synth. Commun. 22, 421 - 428 (1992) dargestellt werden können. Eine Darstellungsmethode aus 2-Ketoanilinen wird von Jones et al. (J. Org. Chem 37, 3622 [1972]) beschrieben.

Ausgangsmaterialien zur Herstellung speziell substituierter Indol-2-carbonsäuren können auch Indol-2-carbonsäuren selbst bilden, an denen in literaturbekannter Weise leicht Substitutionsreaktionen durchgeführt werden. Besonders selektiv können dabei zahlreiche elektrophile Substitutionsreaktionen in 3-Position, aber auch in den restlichen Positionen durchgeführt werden. So verlaufen beispielsweise Halogenierungen mit einen Hal⁺-Überträger glatt und in vollständiger Umsetzung. Am Stickstoffatom in 1-Position können ebenfalls sehr leicht Alkylierungs- und Arylierungsreaktionen durchgeführt werden.

Die Indolin-2-carbonsäuren bzw. deren Ester können generell in literaturbekannter Weise aus den zugrundeliegenden Indol-2-carbonsäuren bzw. deren Estern durch Reduktion bzw. Hydrierung gewonnen werden. Als besonders geeignet erwies sich die Behandlung mit metallischem Magnesium in Methanol analog der Arbeitsanweisung von In Kwon Youn, Gyu Hwan Yon, Chwang Siek Pak; Tetrahedron Letters 27 (1986) 2409-2410.

Benzoylguanidine I sind im allgemeinen schwache Basen und können Säure unter Bildung von Salzen binden. Als Säureadditionssalze kommen Salze aller pharmakologisch verträglichen Säuren in Frage, beispielsweise Halogenide, insbesondere Hydrochloride, Lactate, Sulfate, Citrate, Tartrate, Acetate, Phosphate, Sulfonsäuresalze wie Methylsulfonate, p-Toluolsulfonate usw..

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.
Welche Hilfsstoffe für die gewünschte Arzneimittelformulierung geeignet sind, ist dem Fachmann auf Grund seines Fachwissens geläufig. Neben Lösemitteln, Gelbildnern, Suppositoriengrundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes des Formel I in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg, vorzugsweise 0,01 mg bis höchstens 10 mg, vorzugsweise höchstens 1 mg. Bei akuten Ausbrüchen der Krankheit, etwa unmittelbar nach Erleiden des Herzinfarkts, können auch noch höhere und vor allem häufigere Dosierungen notwendig sein, z. B. bis zu 4 Einzeldosen pro Tag. Insbesondere bei i. v. Anwendung, etwa bei einem Infarktpatienten auf der Intensivstation können bis zu 100 mg pro Tag notwendig werden.

### Beispiele

Allgemeine Vorschrift I zur Herstellung von heterocyclischen Acyl-guanidinen (I) aus heterocyclischen Carbonsäuren (II, L = OH)
0,01 M des Carbonsäurederivates der Formel II (L = OH) löst bzw. suspendiert man in 60 ml wasserfreiem Tetrahydrofuran (THF) und versetzt sodann mit 1,78 g (0,011 M) Carbonyldiimidazol. Nach dem Rühren für 2 Stunden bei Raumtemperatur werden 2,95 g (0,05 M) Guanidin in die Reaktionslösung eingetragen. Nach dem Rühren über Nacht destilliert man das THF unter vermindertem Druck (Rotationsverdampfer) ab, versetzt mit Wasser, stellt mit 2N HCl auf pH 6-7 und filtriert das entsprechende Acylguanidin (Formel I) ab.

Die so erhaltenen heterocyclischen Acylguanidine können durch Behandeln mit wäßriger oder methanolischer Salzsäure oder anderen pharmakologisch verträglichen Säuren in die entsprechenden Salze übergeführt werden.

Allgemeine Vorschrift II zur Herstellung von heterocyclischen Acyl-guanidinen (I) aus den entsprechenden Carbonsäureestern (Formel II, L = Alkoxy oder Phenoxy)

1 Äquivalent eines entsprechenden Carbonsäureesters der Formel II sowie 5.0 Äquivalenten Guanidin werden in Isopropanol gelöst oder in THF suspendiert und bis zur vollständigen Umsetzung (TLC-Kontrolle) am Rückflußkühler gekocht (Reaktionszeit etwa 2 - 8 Stunden). Das Lösungsmittel wird unter vermindertem Druck (Rotationsverdampfer) abdestilliert, der Rückstand in Ethylacetat aufgenommen und 3 mal mit gesättigter Natriumhydrogencarbonat-Lösung gewaschen.
Trocknung der organischen Phase über Natriumsulfat, Abdestillieren des Lösungsmittels unter vermindertem Druck und Chromatographie des Rückstands an Kieselgel unter Anwendung eines geeigneten Laufmittels.
Überführung des erhaltenen entsprechenden heterocyclischen Acyl-guanidins der Formel I in das korrespondierende Hydrochlorid erfolgt analog Vorschrift I.

Entsprechend der allgemeinen Vorschriften I und II wurden die folgenden erfindungsgemäßen Verbindungen hergestellt:

### Beispiel 1:

2-Benzofurylcarbonyl-guanidin-hydrochlorid
Fp. 279 - 283°C.

### Beispiel 2:

6-Chlor-2-benzofurylcarbonyl-guanidin-hydrochlorid,
Fp. 272 - 274°C.

### Beispiel 3:

7-Methoxy-2-benzofurylcarbonyl-guanidin-hydrochlorid,
Fp. 266°C.

### Beispiel 4:

7-Brom-4-hydroxy-3-benzofurylcarbonyl-guanidin-hydrochlorid,
Fp. 239°C.

### Beispiel 5:

7-Hydroxy-2-benzofurylcarbonyl-guanidin-hydrochlorid,
Fp. 243-44°C.

### Beispiel 6:

5-Dimethylaminomethyl-2-benzofurylcarbonyl-guanidin-dihydrochlorid,
Fp. > 250°C.

### Beispiel 7:

5,6-Dichlor-2-benzofurylcarbonyl-guanidin-hydrochlorid,
Fp. > 250°C.

### Beispiel 8:

7-Amino-2-benzthiazolylcarbonyl-guanidin-dihydrochlorid,
Fp. > 250°C.

### Beispiel 9:

2-Amino-6-benzthiazolylcarbonyl-guanidin-dihydrochlorid,
Fp. > 250°C.

### Beispiel 10:

6-Chlor-3-methoxybenzo[b]thien-2-ylcarbonyl-guanidin-hydrochlorid,
Fp. 202°C.

### Beispiel 11:

Benzotriazol-5-ylcarbonyl-guanidin-hydrochlorid,
Fp. 270°C.

### Beispiel 12:

2-Benzthiazolylcarbonyl-guanidin-hydrochlorid,
Fp. 273 - 275°C

### Beispiel 13:

Benzo[b]thien-2-ylcarbonyl-guanidin-hydrochlorid,
Fp. 296 - 298°C.

### Beispiel 14:

2-Chlor-5-methylbenzo[b]thien-2-ylcarbonyl-guanidin-hydrochlorid,
Fp. 221 - 222°C.

### Beispiel 15:

5-Nitrobenzo[b]thien-2-ylcarbonyl-guanidin-hydrochlorid,
Fp. 285°C.

### Beispiel 16:

5-Aminobenzo[b]thien-2-ylcarbonyl-guanidin-hydrochlorid,
Fp. 250°C (Zersetzung).

### Beispiel 17:

1H-Benzimidazol-5-yl-carbonyl-guanidin-hydrochlorid,
Fp. 265°C

### Beispiel 18:

1-(2-Chlorbenzyl)-1H-benzimidazol-5-yl-carbonyl-guanidin-hydrochlorid,
Fp. 265°C.

### Beispiel 19:

1-(1-Hexyl)-1H-benzimidazol-5-yl-carbonyl-guanidin-hydrochlorid,
Fp. 232 - 234°C.

### Beispiel 20:

1-(2-Chlorbenzyl)-2-hydroxy-1H-benzimidazol-5-yl-carbonyl-guanidin-hydrochlorid,
Fp. 283°C.

### Beispiel 21:

2-Methylthio-1H-benzimidazol-5-yl-carbonyl-guanidin-hydrochlorid,
Fp. 211°C.

### Beispiel 22:

1-(2-Chlorbenzyl)-2-methylthio-1H-benzimidazol-5-yl-carbonyl-guanidin-
hydrochlorid, Fp. 220°C.

### Beispiel 23:

1-(1-Hexyl)-2-hydroxy-1H-benzimidazol-5-yl-carbonyl-guanidin-hydrochlorid,
Fp. 227 - 229°C.

### Beispiel 24:

6-[N-Methyl-N-(2-phenylethyl)sulfamoyl]-1H-benzimidazol-4-yl-carbonyl-guanidin-
hydrochlorid, Fp. 205°C.

### Beispiel 25:

1-Methyl-2-methylthio-1H-benzimidazol-6-yl-carbonyl-guanidin-hydrochlorid,
Fp. 246°C.

### Beispiel 26:

6-Sulfamoyl-1H-benzimidazol-4-yl-carbonyl-guanidin-hydrochlorid,
Fp. 223°C.

### Beispiel 27:

2-(2-Phenylethyl)-6-N-pyrrolidinosulfonyl-1H-benzimidazol-4-yl-carbonyl-guanidin-hydrochlorid, Fp. 192°C (Zersetzung).

### Beispiel 28:

6-Chlor-2-(2-phenylethyl)-1H-benzimidazol-4-yl-carbonyl-guanidin-hydrochlorid,
Fp. 262 - 264°C.

### Beispiel 29:

6-N-Pyrrolidinosulfonyl-1H-benzimidazol-4-yl-carbonyl-guanidin-hydrochlorid,
Fp. 200 - 202°C (Zersetzung).

### Beispiel 30:

6-Methylsulfonyl-2-(2-phenylethyl)-1H-benzimidazol-4-yl-carbonyl-guanidin-
hydrochlorid, Fp. 215°C.

### Beispiel 31:

6-[N-Methyl-N-(2-phenylethyl)sulfamoyl]-2-(2-phenylethyl)-1H-benzimidazol-4-yl-carbonyl-guanidin-hydrochlorid, Fp. 130°C.

### Beispiel 32:

2-Benzylthio-6-methylsulfonyl-1H-benzimidazol-4-yl-carbonyl-guanidin-hydrochlorid,
Fp. 215°C.

### Beispiel 33:

5-Chlor-1-methyl-1H-benzimidazol-2-yl-carbonyl-guanidin-hydrochlorid,
Fp. 228°C.

### Beispiel 34:

5,6-Dichlor-1-methyl-1H-benzimidazol-2-yl-carbonyl-guanidin-hydrochlorid,
Fp. 274°C. (Zersetzung)

### Beispiel 35:

3-Indolylcarbonyl-guanidin-hydrochlorid,
Fp. 270°C. (Zersetzung)

### Beispiel 36:

5-Indolylcarbonyl-guanidin-hydrochlorid,
Fp. 258°C. (Zersetzung)

### Beispiel 37:

1-Benzyl-3-indolylcarbonyl-guanidin-hydrochlorid,
Fp. 256°C. (Zersetzung)

### Beispiel 38:

4-Indolylcarbonyl-guanidin-hydrochlorid,
Fp. 270°C. (Zersetzung)

### Beispiel 39:

1-Methyl-3-indolylcarbonyl-guanidin-hydrochlorid,
Fp. 250°C. (Zersetzung)

### Beispiel 40:

1-(2-N,N-Dimethylaminoethyl)-3-indolylcarbonyl-guanidin-dihydrochlorid,
Fp. 250°C. (Zersetzung)

### Beispiel 41:

1-Methyl-2-indolylcarbonyl-guanidin-hydrochlorid,
Fp. 280°C.

### Beispiel 42:

2-Indolylcarbonyl-guanidin-hydrochlorid,
Fp. 310 - 312°C.

### Beispiel 43:

5-Chlor-2-indolylcarbonyl-guanidin-hydrochlorid,
Fp. 317 - 320°C.

### Beispiel 44:

5-Methoxy-2-indolylcarbonyl-guanidin-hydrochlorid,
Fp. 292°C.

### Beispiel 45:

5-Chlor-1-methyl-2-indolylcarbonyl-guanidin-hydrochlorid,
Fp. 269°C.

### Beispiel 46:

1-(3,4-Dichlorbenzyl)-2-indolylcarbonyl-guanidin-hydrochlorid,
Fp. 195°C.

### Beispiel 47:

1-(2-N,N-Dimethylaminoethyl)-2-indolylcarbonyl-guanidin-dihydrochlorid,
Fp. 255°C. (Zersetzung)

### Beispiel 48:

1-Benzyl-2-indolylcarbonyl-guanidin-hydrochlorid,
Fp. 229°C.

### Beispiel 49:

1-Ethyl-2-indolylcarbonyl-guanidin-hydrochlorid,
Fp. 200 - 201°C.

### Beispiel 50:

1-Benzyl-5-chlor-2-indolylcarbonyl-guanidin-hydrochlorid,
Fp. 198°C.

### Beispiel 51:

5-Chlor-1-(2-N,N-dimethylaminoethyl)-2-indolylcarbonyl-guanidin-dihydrochlorid,
Fp. 255°C. (Zersetzung)

### Beispiel 52:

5-Chlor-1-ethyl-2-indolylcarbonyl-guanidin-hydrochlorid,
Fp. 255°C.

### Beispiel 53:

5-Chlor-1-propyl-2-indolylcarbonyl-guanidin-hydrochlorid,
Fp. 255°C.

### Beispiel 54:

5-Chlor-1-butyl-2-indolylcarbonyl-guanidin-hydrochlorid,
Fp. 222°C.

### Beispiel 55:

5-Hydroxy-1-methyl-2-indolylcarbonyl-guanidin-hydrochlorid,
Fp. 288°C.

### Beispiel 56:

3,5-Dichlor-1-(2-N,N-dimethylaminoethyl)-2-indolylcarbonyl-guanidin-dihydrochlorid,
Fp. 246°C.

### Beispiel 57:

5-Methoxy-1-methyl-2-indolylcarbonyl-guanidin-hydrochlorid,
Fp. 227°C.

### Beispiel 58:

3-Chlor-1-methyl-2-indolylcarbonyl-guanidin-hydrochlorid,
Fp. 216°C.

### Beispiel 59:

3-Chlor-2-indolylcarbonyl-guanidin-hydrochlorid,
Fp. 222°C.

### Beispiel 60:

3,5-Dichlor-1-(4-picolyl)-2-indolylcarbonyl-guanidin-dihydrochlorid,
Fp. 220°C.

### Beispiel 61:

5-Chlor-1-(4-picolyl)-2-indolylcarbonyl-guanidin-dihydrochlorid,
Fp. 287°C.

### Beispiel 62:

5-Chlor-1,3-dimethyl-2-indolylcarbonyl-guanidin-hydrochlorid,
Fp. 258°C.

### Beispiel 63:

5,7-Dichlor-1,3-dimethyl-2-indolylcarbonyl-guanidin-hydrochlorid,
Fp. 307°C.

### Beispiel 64:

4,6-Dichlor-1,3-dimethyl-2-indolylcarbonyl-guanidin-hydrochlorid,
Fp. 296°C.

### Beispiel 65:

5-Chlor-1-methyl-3-phenyl-2-indolylcarbonyl-guanidin-hydrochlorid,
Fp. 288°C.

### Beispiel 66:

3-Brom-1-methyl-2-indolylcarbonyl-guanidin-hydrochlorid,
Fp. 208 - 210°C.

### Beispiel 67:

5-Fluor-1-methyl-2-indolylcarbonyl-guanidin-hydrochlorid,
Fp. 278°C.

### Beispiel 68:

3,5-Dichlor-2-indolylcarbonyl-guanidin-hydrochlorid,
Fp. 208 - 210°C.

### Beispiel 69:

3,5-Dichlor-1-methyl-2-indolylcarbonyl-guanidin-hydrochlorid,
Fp. 208 - 210°C.

### Beispiel 70:

4,5,6-Trichlor-1,3-dimethyl-2-indolylcarbonyl-guanidin-hydrochlorid,
Fp. 307°C.

### Beispiel 71:

2-(4-Methoxyphenyl)-benzimidazol-5-carbonsäure-guanidid
Methansulfonsäuresalz; Fp. 270°C

### Beispiel 72:

2-(4-Trifluormethylphenyl)-benzimidazol-5-carbonsäureguanidid
Methansulfonsäuresalz; Fp. 275 °C

### Beispiel 73:

2-Phenylbenzimidazol-4-carbonsäureguanidid
Fp. 264°C

### Beispiel 74:

2-(4-Fluorphenyl)-1-methylbenzimidazol-5-carbonsäureguanidid
Methansulfonsäuresalz; Fp. 252°C

### Beispiel 75:

2-(4-Methylphenyl)-benzimidazol-5-carbonsäureguanidid Methansulfonsäuresalz;
Fp. 285°C

### Beispiel 76:

2-(4-Hydroxyphenyl)-benzimidazol-4-carbonsäureguanidid
Methansulfonsäuresalz; Fp. 281°C

### Beispiel 77:

2-[4-(4-Chlorphenoxy)phenyl]-benzimidazol-5-carbonsäureguanidid
Methansulfonsäuresalz; Fp. 239°C

### Beispiel 78:

2-Benzylbenzimidazol-5-carbonsäureguanidid Methansulfonsäuresalz; Fp. 210°C

### Beispiel 79:

2-Undecylbenzimidazol-5-carbonsäureguanidid Methansulfonsäuresalz; Fp. 252°C

### Beispiel 80:

2-Methylbenzimidazol-5-carbonsäureguanidid Methansulfonsäuresalz; Fp. 279°C

### Beispiel 81:

1-Methyl-2-phenylbenzimidazol-5-carbonsäureguanidid Methansulfonsäuresalz;
Zersetzungspunkt: 230°C

### Beispiel 82:

1-Methyl-2-(2-thienyl)benzimidazol-5-carbonsäureguanidid
Methansulfonsäuresalz; Fp. 211°C

### Beispiel 83:

2-Chlor-1-phenylindol-3-carbonsäureguanidid Methansulfonsäuresalz; Fp. 203 - 205°C

### Beispiel 84:

2-Phenoxy-1-phenylindol-3-carbonsäureguanidid Methansulfonsäuresalz; Fp. 202 - 204°C

### Beispiel 85:

5-Chlor-1-ethylbenzimidazol-2-carbonsäureguanidid Hydrochlorid;
Fp: 244°C.

### Beispiel 86:

3-Chlor-5-fluor-1-methylindolyl-2-carbonsäureguanidid Hydrochlorid; Fp: 234°C.

### Beispiel 87:

5-Methoxy-1-phenylindolyl-2-carbonsäureguanidid Hydrochlorid;
Fp: 266°C.

### Beispiel 88:

3-Isopropyl-5-methoxyindolyl-2-carbonsäureguanidid Hydrochlorid;
Fp: 210°C.

### Beispiel 89:

5-Chlor-3-phenylthio-2-indolylcarbonsäureguanidid Hydrochlorid;
Fp: 252°C.

### Beispiel 90:

3,5-Dimethoxy-1-methyl-2-indolylcarbonsäureguanidid Methansulfonsäuresalz;
Fp. 212°C

### Beispiel 91:

3-Isopropyloxy-5-methoxy-1-methyl-2-indolylcarbonsäureguanidid Methansulfonsäuresalz;
Fp. 248°C

### Beispiel 92:

5-Chlor-3-phenylsulfonyl-2-indolylcarbonsäureguanidid Methansulfonsäuresalz;
Fp. 240°C

### Beispiel 93:

5-Chlor-1-phenyl-2-indolylcarbonsäureguanidid Methansulfonsäuresalz;
Zersetzungspunkt: 305°C

### Beispiel 94:

5-Chlor-3-isopropyl-2-indolylcarbonsäureguanidid Methansulfonsäuresalz;
Zersetzungspunkt: 258 - 259°C

### Beispiel 95:

5-Chlor-3-methoxy-1-methyl-2-indolylcarbonsäureguanidid Methansulfonsäuresalz;
Zersetzungspunkt: 234°C

### Beispiel 96:

5-Chlor-3-isopropyl-1-methyl-2-indolylcarbonsäureguanidid Methansulfonsäuresalz;
Zersetzungspunkt: 164 - 166°C

### Beispiel 97:

5-Trifluormethyl-3-methyl-2-indolylcarbonsäureguanidid Hydrochlorid;
Zersetzungspunkt: 232 - 236°C

### Beispiel 98:

5-Trifluormethyl-1,3-dimethyl-2-indolylcarbonsäureguanidid Hydrochlorid;
Zersetzungspunkt: 219 - 223°C

### Beispiel 99:

5-Benzoyl-1,3-dimethyl-2-indolylcarbonsäureguanidid Hydrochlorid;
Fp. 270 - 273°C

### Beispiel 100:

5-Cyclohexyl-1,3-dimethyl-2-indolylcarbonsäureguanidid Hydrochlorid;
Fp. 191 - 194°C

### Beispiel 101:

1,3,5-Trimethyl-2-indolylcarbonsäureguanidid Hydrochlorid;
Fp. 210 - 212°C

### Beispiel 102:

1,3,4,6-Tetramethyl-2-indolylcarbonsäureguanidid Hydrochlorid;
Zersetzungspunkt: 164 - 171°C

### Beispiel 103:

5-Methylsulfonyl-2-indolylcarbonsäureguanidid Hydrochlorid;
Fp. 298 - 305°C

### Beispiel 104:

1-Methylindolin-2-carbonsäure-guanidid-hydrochlorid:
a ) Indolin-2-carbonsäure wurde in DMF mit 2 eq. K₂CO₃ und Methyliodid zum N-Methyl-indolin-2-carbonsäuremethylester umgesetzt.
   Farbloses Öl
   MS (ES): 192 (M+1)

Der Ester aus a ) wurde mit 5 eq. Guanidin in THF unter Rückfluß erhitzt. Das Lösungsmittel wurde im Vakuum abgezogen, der Rückstand in Wasser aufgenommen und dreimal mit EE extrahiert. Die vereinigten organischen Phasen wurden über MgSO₄ getrocknet und das resultierende Guanidid durch Zusatz von methanolischer HCl als Hydrochlorid isoliert.
Feststoff, Fp 162 - 170°C.

### Beispiel 105:

1-Methyl-(S)-indolin-2-carbonsäure-guanidid Hydrochlorid
Wurde in analoger Weise aus (S)-Indolin-2-carbonsäure hergestellt.
Feststoff, Fp 162°C.

### Beispiel 106

5-Fluor-1-methylindolin-2-carbonsäureguanidid Hydrochlorid
a) 5-Fluor-indol-2-carbonsäuremethylester wurde nach einer literaturbekannten Vorschrift [In Kwon Youn, Gyu Hwan Yon, Chwang Siek Pak; Tetrahedron Letters 27 (1986) 2409-2410] mit Magnesium in Methanol zum 5-Fluor-indolin-2-carbonsäuremethylester umgesetzt.
   Farbloses Öl,
   MS (ES): 196 (M+1)
b) Der Indolin-2-carbonsäureester aus Reaktion a) wurde in DMF mit K₂CO₃ und Methyliodid N-methyliert. Nach Standardaufarbeitung isolierte man N-Methyl-5-fluor-indolin-2-carbonsäuremethylester.
Farbloses Öl,
   MS (ES): 210 (M+1)
c) Das Produkt aus Reaktion b) wurde mit 5 eq. Guanidin in THF unter Rückfluß erhitzt. Das Lösungsmittel wurde im Vakuum abgezogen, der Rückstand in Wasser aufgenommen und dreimal mit EE extrahiert. Die vereinigten organischen Phasen wurden über MgSO₄ getrocknet und das resultierende Guanidid durch Zusatz von methanolischer HCl als Hydrochlorid isoliert.
   Rötlicher Feststoff, Fp 135 - 150°C.

### Beispiel 107

5-Methoxy-1-methylindolin-2-carbonsäureguanidid Hydrochlorid Wurde in analoger Weise dargestellt.
Rötlicher Feststoff, Fp 169 - 174°C.

Diese Transformation von Indol-2-carbonsäurederivaten in die entsprechenden Indolin-derivate in den Beispielen 104 bis 107 ist ebenfalls auf alle anderen Indol-carbonsäuren übertragbar.

### Beispiel 108

1-Methylindol-2-carbonsäure-guanidid
erhält man durch Behandlung einer Suspension von 1,5 g 1-Methylindol-2-carbonsäure-guanidid Hydrochlorid in 30 ml Wasser mit wäßriger Natriumbicarbonat-Lösung. Farblose Kristalle, Fp. 95 - 100°C.

### Darstellungsweisen von Vorstufen

### A. 3-Jodindol-2-carbonsäurederivate

erhält man beispielsweise durch Zutropfen einer Lösung von 0,02 Mol Jodmonochlorid in Eisessig zu einer Lösung oder Suspension von 0,02 Mol Indol-2-carbonsäureester bei Raumtemperatur, anschließendem Rühren für 30 Minuten bei 40°C. Nach dem Abdestillieren des Lösungsmittels wird der Rückstand mit Wasser aufgenommen und der kristalline Feststoff abfiltriert.

5-Chlor-3-jodindol-2-carbonsäureethylester: Farblose Kristalle, Fp. 190-194°C.
3-Jodindol-2-carbonsäureethylester: Farblose Kristalle, Fp. 127-131°C
5-Benzyloxy-3-jodindol-2-carbonsäureethylester: Farblose Kristalle, Fp. 122 - 124°C
5-Fluor-3-jodindol-2-carbonsäureethylester: Farblose Kristalle, Fp. 154 - 158°C.

### B. 3-Bromindol-2-carbonsäurederivate

erhält man beispielsweise durch Zutropfen einer Lösung von 0,05 Mol Brom in Eisessig zu einer Lösung oder Suspension von 0,05 Mol Indol-2-carbonsäureester bei Raumtemperatur, anschließendem Rühren für weitere 15 Minuten bei 40°C. Nach dem Abdestillieren des Lösungsmittels wird der Rückstand mit Wasser aufgenommen, der kristalline Feststoff abfiltriert und der 3-Bromindol-2-carbonsäureester durch Umkristallisation gereinigt.

### Beispiel B1:

3-Bromindol-2-carbonsäureethylester: Farblose Kristalle, Fp. 143 - 144°C.

### Beispiel B2:

3-Brom-5-chlorindol-2-carbonsäureethylester: Farblose Kristalle, Fp. 184 - 186°C.

### C. 3-Chlorindol-2-carbonsäurederivate

erhält man beispielsweise durch Zutropfen einer Lösung von 0,05 Mol N-Chlorsuccinimid in Eisessig zu einer Lösung oder Suspension von 0,05 Mol Indol-2-carbonsäureester bei Raumtemperatur, anschließendem Rühren für weitere 15 Minuten bei 40°C und anschließendem Nachrühren bei Raumtemperatur. Nach dem Abdestillieren des Lösungsmittels wird der Rückstand mit Wasser aufgenommen und mit gesättigter wäßriger Natriumbicarbonatlösung auf pH 8 gestellt. Der kristalline Feststoff wird abfiltriert und der 3-Chlorindol-2-carbonsäureester durch Umkristallisation gereinigt.

### Beispiel C1):

3,5-Dichlorindol-2-carbonsäureethylester; Farblose Kristalle, Fp. 163 - 165°C.

### Beispiel C2):

3-Chlor-5-fluorindol-2-carbonsäureethylester; Farblose Kristalle, Fp. 138 - 141°C.

### Beispiel C3):

3-Brom-5-chlorindol-2-carbonsäureethylester, kristalliner Feststoff, Fp. 178 - 182°C.

### D. 1-Alkylierungen von 2-Indolcarbonyl-Derivaten

führt man beispielsweise in der Weise durch, daß man eine Mischung aus 0,05 Mol des jeweiligen Indol-2-carbonsäureesters, 0,06 Mol Alkylierungsmittel und 0,015 Mol feingepulvertes, wasserfreies Kaliumcarbonat in 10 bis 50 ml Aceton ca 5 - 8 Stunden am Rückflußkühler kocht und den Reaktionsfortgang dünnschichtchromatografisch verfolgt. Anschließend wird das Lösungsmittel abdestilliert, der Rückstand mit Wasser versetzt und mit Essigsäureethylester extrahiert.

### Beispiel D1):

3-Jod-1-methylindol-2-carbonsäureethylester:
Alkylierungsmittel: Methyljodid;
Eigenschaften: Hellgelbes Öl.

### Beispiel D2):

5-Chlor-3-jod-1-methylindol-2-carbonsäureethylester:
Alkylierungsmittel: Methyljodid; Eigenschaften: Farblose kristalline Substanz, Fp. 88 - 93°C

### Beispiel D3):

3-Jod-1-(2-dimethylaminoethyl)indol-2-carbonsäureethylester:
Alkylierungsmittel: 2-Dimethylaminoethylchlorid Hydrochlorid;
Eigenschaften: Hellgelbes Öl.

### Beispiel D4):

3-Jod-1-(3,4-dichlorbenzyl)indol-2-carbonsäureethylester:
Alkylierungsmittel: 3,4-Dichlorbenzylchlorid;
Eigenschaften: Hellgelbes Öl.

### Beispiel D5):

5-Chlor-1-ethyl-3-jodindol-2-carbonsäureethylester:
Alkylierungsmittel: Ethylbromid
Eigenschaften: Farbloser, kristalliner Feststoff, Fp. 56 - 59°C.

### Beispiel D6):

5-Chlor-3-jod-1-propylindol-2-carbonsäureethylester:
Alkylierungsmittel: Propyljodid
Eigenschaften: Farbloser, kristalliner Feststoff, Fp. 77 - 84°C.

### Beispiel D7):

1-Butyl-5-chlor-3-jodindol-2-carbonsäureethylester:
Alkylierungsmittel: Butyljodid
Eigenschaften: Hellgelbes Öl.

### Beispiel D8):

5-Chlor-3-jod-1-isopropylindol-2-carbonsäureethylester:
Alkylierungsmittel: Isopropyljodid
Eigenschaften: Farbloser, kristalliner Feststoff, Fp. 162 - 166°C.

### Beispiel D9):

5-Benzyloxy-3-jod-1-methylindol-2-carbonsäureethylester:
Alkylierungsmittel: Methyljodid
Eigenschaften: Farbloser, kristalliner Feststoff, unscharfer Fp. 90 - 105°C.

### Beispiel D10):

5-Fluor-3-jod-1-methylindol-2-carbonsäureethylester:
Alkylierungsmittel: Methyljodid
Eigenschaften: Farbloser, kristalliner Feststoff, unscharfer Fp. 76 - 79°C.

### Beispiel D11):

3,5-Dichlor-1-methylindol-2-carbonsäureethylester:
Alkylierungsmittel: Methyljodid
Eigenschaften: Farbloser, kristalliner Feststoff, unscharfer Fp. 54 - 56°C.

### Beispiel D12):

3,5-Dichlor-1-(2-dimethylaminoethyl)indol-2-carbonsäureethylester:
Alkylierungsmittel: 2-Dimethylaminoethylchlorid Hydrochlorid
Eigenschaften: Öl.

### Beispiel D13)

3,5-Dichlor-1-(4-picolyl)indol-2-carbonsäureethylester:
Alkylierungsmittel: 4-Picolylchlorid-hydrochlorid
Eigenschaften: Fp. 100 - 103°C.

### Beispiel D14)

5-Chlor-3-jod-1-(4-picolyl)indol-2-carbonsäureethylester:
Alkylierungsmittel: 4-Picolylchlorid-hydrochlorid
Eigenschaften: Fp. 127 - 129°C.

### Beispiel D15)

2-Acetyl-5-chlor-1,3-dimethylindol
Alkylierungsmittel: Methyljodid
Eigenschaften: Fp 88°C.

### Beispiel D16)

2-Acetyl-5-chlor-1-methyl-3-phenylindol
Alkylierungsmittel: Methyljodid
Eigenschaften: Fp. 89 - 91°C.

### Beispiel D17)

2-Acetyl-5,7-dichlor-1,3-dimethylindol
Alkylierungsmittel: Methyljodid
Eigenschaften: Fp 105 - 108°C.

### Beispiel D18)

2-Acetyl-4,6-dichlor-1,3-dimethylindol
Alkylierungsmittel: Methyljodid
Eigenschaften: Fp. 115 - 118°C.

### Beispiel D19)

2-Acetyl-4,5,6-trichlor-1,3-dimethylindol
Alkylierungsmittel: Methyljodid
Eigenschaften: Fp. 129°C.

### Beispiel D20)

5-Fluor-3-chlor-1-methylindol-2-carbonsäureethylester:
Alkylierungsmittel: Methyljodid
Eigenschaften: Farbloser, kristalliner Feststoff, unscharfer Fp.65 - 69°C.

### Beispiel D21)

5-Chlor-3-phenylsulfonyl-1-methylindol-2-carbonsäure-methylester durch Methylierung von 5-Chlor-3-phenylsulfonylindol-2-carbonsäure
Alkylierungsmittel: Methyljodid
Eigenschaften: Farbloser, kristalliner Feststoff, Fp. 192 - 196°C.

### Beispiel D22)

3-Brom-5-chlor-1-methylindol-2-carbonsäure-ethylester durch Alkylierungsmittel:
Methyljodid
Eigenschaften: Farbloser, kristalliner Feststoff, Fp. 60 - 63°C.

### Beispiel D23)

2-Acetyl-5-isopropyl-1,3-dimethylindol
Alkylierungsmittel: Methyljodid
Eigenschaften: Farbloser, kristalliner Feststoff, Fp. 74 - 77°C.

### Beispiel D24)

2-Acetyl-5-benzoyl-1,3-dimethylindol
Alkylierungsmittel: Methyljodid
Eigenschaften: Farbloser, kristalliner Feststoff, Fp. 113°C.

### Beispiel D25)

2-Acetyl-5-benzoyl-1,3,5-trimethylindol
Alkylierungsmittel: Methyljodid
Eigenschaften: Farbloser, kristalliner Feststoff, Fp. 175°C.

### E. Darstellung von 1-Alkylcarbonsäure-Derivaten durch dehalogenierende Hydrogenierung

wurden bevorzugt mit 3-Jod- oder 3-Bromindol-2-carbonsäure-Derivaten durchgeführt:
0,005 Mol des 3-Halogenindol-2-carbonsäureesters werden in Methanol mit 300 mg Palladium auf Kohle (10%) bis zum Erreichen der theoretischen Wasserstoffaufnahme unter Schütteln bei Raumtemperatur hydriert, das Lösungsmittel abdestilliert und der Rückstand mit Wasser versetzt.

### Beispiel E1):

5-Chlor-1-methylindol-2-carbonsäureethylester
Eigenschaften: Farblose, kristalline Substanz, Fp. 68 - 72°C.

### Beispiel E2):

1-(2-Dimethylaminoethyl)indol-2-carbonsäureethylester
Eigenschaften: Hellgelbes Öl.

### Beispiel E3):

1-(3,4-Dichlorbenzyl)indol-2-carbonsäureethylester
Eigenschaften: Farbloser, kristalliner Feststoff; Fp. 88 - 92°C

### Beispiel E4):

5-Chlor-1-ethylindol-2-carbonsäureethylester
Eigenschaften: Farblose, kristalline Substanz, Fp. 53 - 56°C.

### Beispiel E5):

1-Isopropyl-5-chlorindol-2-carbonsäureethylester
Eigenschaften: Farblose, kristalline Substanz, Fp. 151 - 155°C.

### Beispiel E6):

5-Chlor-1-propylindol-2-carbonsäureethylester
Eigenschaften: Farblose, kristalline Substanz, Fp. 49 - 54°C.

### Beispiel E7):

1-Butyl-5-chlorindol-2-carbonsäureethylester
Eigenschaften: Öl.

### Beispiel E8):

5-Benzyloxy-1-methylindol-2-carbonsäureethylester
Eigenschaften: Farblose, kristalline Substanz, Fp. 120 - 127°C.

### Beispiel E9):

5-Fluor-1-methylindol-2-carbonsäureethylester
Eigenschaften: Farblose, kristalline Substanz, Fp. 68 - 69°C.

### Beispiel E10:

5-Chlor-1-(4-picolyl)indol-2-carbonsäureethylester
Eigenschaften: Fp. 76 - 78°C.

### F. Hydrolyse von Indol-2-carbonsäurestern

führt man beispielsweise durch Erhitzen des Indol-2-carbonsäuresters in einem Gemisch aus Wasser und Methanol mit etwa 3 Mol NaOH bis zur Auflösung der Suspension durch. Aufarbeitung nach Sauerstellen mit 2 N Salzsäure auf pH 1 bis 2 und Extraktion oder Kristallisation.

### Beispiel F1):

5-Chlor-1-methylindol-2-carbonsäure
Eigenschaften: Zersetzung bei 235 - 239°C.

### Beispiel F2):

1-(2-Dimethylaminoethyl)indol-2-carbonsäure;
Hinweis: Isolierung durch Kristallisation aus wenig Wasser als inneres Salz bei pH 4 - 5.
Eigenschaften: Farblose kristalline Substanz, Fp. 214 - 216°C.

### Beispiel F3):

1-(3,4-Dichlorbenzyl)indol-2-carbonsäure
Eigenschaften: Farbloser, kristalliner Feststoff; Fp. 152 - 155°C

### Beispiel F4):

5-Chlor-1-ethylindol-2-carbonsäure
Eigenschaften: Farbloser, kristalliner Feststoff; Fp. 194 - 200°C.

### Beispiel F5):

5-Chlor-1-propylindol-2-carbonsäure
Eigenschaften: Farbloser, kristalliner Feststoff; Fp. 152 - 154°C.

### Beispiel F6):

1-Butyl-5-chlorindol-2-carbonsäure
Eigenschaften: Farbloser, kristalliner Feststoff; Fp. 175°C.

### Beispiel F7):

5-Benzyloxy-1-methylindol-2-carbonsäure
Eigenschaften: Farbloser, kristalliner Feststoff; Fp. 218 - 222°C.

### Beispiel F8):

5-Fluor-1-methylindol-2-carbonsäure
Eigenschaften: Farbloser, kristalliner Feststoff; Fp. 227°C.

### Beispiel F9):

3,5-Dichlorindol-2-carbonsäure
Eigenschaften: Farbloser, kristalliner Feststoff, Fp. 235 - 240°C.

### Beispiel F10):

3,5-Dichlor-1-methylindol-2-carbonsäure
Eigenschaften: Farbloser, kristalliner Feststoff, Fp. 250 - 252°C.

### Beispiel F11):

3,5-Dichlor-1-(2-dimethylaminoethyl)indol-2-carbonsäure
Eigenschaften: Farbloser, kristalliner Feststoff, Fp. 243 - 246°C.

### Beispiel F12:

5-Methoxy-1-methylindol-2-carbonsäure
Eigenschaften: Farbloser, kristalliner Feststoff, Fp. 211 - 214°C.

### Beispiel F13:

3,5-Dichlor-1-(4-picolyl)indol-2-carbonsäure
Eigenschaften: Farbloser, kristalliner Feststoff, Fp. 228 - 232°C.

### Beispiel F14:

5-Chlor-1-(4-picolyl)indol-2-carbonsäure:
Eigenschaften: Fp. 288 - 290°C.

### Beispiel F15:

3-Chlor-5-fluor-1-methylindol-2-carbonsäure
Eigenschaften: Farbloser, kristalliner Feststoff; Fp. 222 - 225°C.

### Beispiel F16:

3-Isopropyl-5-methoxyindol-2-carbonsäure. Kristalliner Feststoff; Fp. 156 -
158°C.

### Beispiel F17:

5-Chlor-3-phenylsulfonyl-1-methylindol-2-carbonsäure
Eigenschaften: Farbloser, kristalliner Feststoff, Fp. 238 - 241°C.

### Beispiel F18:

3-Jodindol-2-carbonsäure
Eigenschaften: Farbloser, kristalliner Feststoff, Fp. 177 - 179°C.

### Beispiel F19:

3-Jod-1-methylindol-2-carbonsäure
Eigenschaften: Farbloser, kristalliner Feststoff, Fp. 177 - 179°C (Zersetzung)

### G. Darstellung von Alkandion-monophenylhydrazonen

Allgemeine Synthesevorschrift:
Eine Lösung aus 0,127 Mol NaOH in 20 ml Wasser wird unter Rührung zu einer Lösung von 0,103 Mol β-Ketocarbonsäureester in 25 ml Ethanol gegeben und 30 Minuten bei Raumtemperatur gerührt. Sodann fügt man 200 ml Wasser zu, rührt weitere 4 Stunden bei Raumtemperatur und extrahiert das Gemisch mit ca. 50 - 100 ml Diethylether.
In die so hergestellte wäßrige Phase gießt man portionsweise eine eiskalte Diazoniumsalz-Lösung, die wie folgt hergestellt wurde:
0,1 Mol der Anilinkomponente werden mit 150 ml 18%iger Salzsäure versetzt und erwärmt, wobei meistens eine dicke Kristallsuspension entsteht. Nach dem Abkühlen tropft man eine Lösung von 0,1 Mol Natriumnitrit unter gutem Rühren so unter die Flüssigkeitsoberfläche, daß die Temperatur zwischen 0 und 5°C gehalten wird und rührt nach der Zugabe weitere 10 Minuten nach. Die so erhaltene, zumeist weitgehend klare Lösung des Diazoniumsalzes gießt man portionsweise zu der alkalischen Lösung des β-Ketocarbonsäureesters. Nach Zugabe von ca. 300 ml Wasser stellt man durch Eintragen von Natriumacetat auf pH 5 und filtriert den kristallinen Niederschlag oder extrahiert bei öligen Niederschlägen mit Essigsäureethylester.

### Beispiel G1)

Anilin: 4-Chloranilin, β-Ketoester: 2-Ethylacetessigsäureethylester
Endprodukt: 2,3-Pentandion-3-N-(4-chlorphenyl)hydrazon; Fp. 153 - 157°C.

### Beispiel G2)

Anilin: 4 -Chloranilin, β-Ketoester: 2-Benzylacetessigsäureethylester
Endprodukt: 1-Phenylbutan-2,3-dion-3-N-(4-chlorphenyl)hydrazon; Fp. 98 - 104°C

### Beispiel G3)

Anilin: 3,5 -Dichloranilin, β-Ketoester: 2-Ethylacetessigsäureethylester
Endprodukt: 2,3-Pentandion-3-N-(3,5-dichlorphenyl)hydrazon; Fp. 175°C

### Beispiel G4)

Anilin: 2,4 -Dichloranilin, β-Ketoester: 2-Ethylacetessigsäureethylester
Endprodukt: 2,3-Pentandion-3-N-(2,4-dichlorphenyl)hydrazon; Fp. 48 - 53°C

### Beispiel G5)

Anilin: 2,3,4 -Trichloranilin, β-Ketoester: 2-Ethylacetessigsäureethylester
Endprodukt: 2,3-Pentandion-3-N-(2,3,4-trichlorphenyl)hydrazon; Fp. 214 - 218°C

### Beispiel G6)

Anilin: 4-Isopropylanilin, β-Ketoester: 2-Ethylacetessigsäureethylester
Endprodukt: 2,3-Pentandion-3-N-(4-isopropylphenyl)hydrazon; Fp. 214 - 218°C

### Beispiel G7)

Anilin: 4-Aminobenzophenon, β-Ketoester: 2-Ethylacetessigsäureethylester
Endprodukt: 2,3-Pentandion-3-N-(4-benzoylphenyl)hydrazon; Fp. 114 - 118°C

### Beispiel G8)

Anilin: p-Toluidin, β-Ketoester: 2-Ethylacetessigsäureethylester
Endprodukt: 2,3-Pentandion-3-N-(4-methylphenyl)hydrazon; Fp. 136°C (Zersetzung)

### Beispiel G9)

Anilin: 3,5-Dimethylanilin, β-Ketoester: 2-Ethylacetessigsäureethylester
Endprodukt: 2,3-Pentandion-3-N-(3,5-dimethylphenyl)hydrazon; Fp. 122°C (Zersetzung)

### Beispiel G10)

Anilin: 4-Trifluormethylanilin, β-Ketoester: 2-Ethylacetessigsäureethylester
Endprodukt: 2,3-Pentandion-3-N-(4-trifluormethylphenyl)hydrazon; Fp. 120°C

### Beispiel G11)

Anilin: 3,5-Bis-trifluormethyl-anilin, β-Ketoester: 2-Ethylacetessigsäureethylester
Endprodukt: 2,3-Pentandion-3-N-(3,5-bis-trifluormethylphenyl)hydrazon
Fp. zieht sich von 110 bis 160°C.

### Beispiel G12)

Anilin: 4-Cyclohexylanilin, β-Ketoester: 2-Ethylacetessigsäureethylester
Endprodukt: 2,3-Pentandion-3-N-(4-cyclohexylphenyl)hydrazon; Fp. 107°C

### H. Darstellung von 2-Acetylindol-Derivaten durch saure Cyclisierung von Phenylhydrazonen (G.) (modifizierte Synthese nach Rajur et al., Synthetic Commun. (1992) 22: 421 - 428)

### Allgemeine Synthesevorschrift:

0,02 Mol eines Phenylhydrazons (siehe G) werden in 70 ml Trifluoressigsäure gelöst, mit einigen Tropfen Trifluormethansulfonsäure versetzt und 5 - 10 Stunden am Rückflußkühler gekocht, und der Reaktionsfortschritt wird dünnschichtchromatografisch verfolgt. Man destilliert das Lösungsmittel ab, versetzt den zumeist dunklen Rückstand mit Wasser und filtriert den kristallinen Niederschlag ab.

### Beispiel H1:

2-Acetyl-5-chlor-3-methylindol; Fp. 172 - 176°C.

### Beispiel H2:

2-Acetyl-5-chlor-3-phenylindol; Fp. 124°C.

### Beispiel H3:

2-Acetyl-4,6-dichlor-3-methylindol; Fp. 178°C (aus wenig Ethanol)

### Beispiel H4:

2-Acetyl-5,7-dichlor-3-methylindol; Fp. 165 - 168°C.

### Beispiel H5:

2-Acetyl-4,5,6-trichlor-3-methylindol; lang gezogener Fp. 188 - 202°C.

### Beispiel H6:

2-Acetyl-5-isopropyl-3-methylindol; Fp. 174 - 177°C.

### Beispiel H7:

2-Acetyl-5-benzoyl-3-methylindol; Fp. 158 - 164°C.

### Beispiel H8:

2-Acetyl-3,5-dimethylindol wurde ohne Isolierung in 1-Stellung methyliert

### Beispiel H9:

2-Acetyl-3,4,6-trimethylindol; Fp. ab 156°C

### Beispiel H10:

2-Acetyl-5-trifluormethyl-3-methylindol; Fp. ab 246°C

### Beispiel H11:

2-Acetyl-5-cyclohexyl-3-methylindol; Fp. ab 209 - 212°C

### Beispiel H12:

2-Acetyl-5-trifluormethyl-3-methylindol; Fp. ab 314°C

### I. Darstellung von Indol-2-carbonsäure-Derivaten aus 2-Acetylindol-Derivaten

### Allgemeine Synthesevorschrift:

Zu einer Lösung aus 12,5 g (0,314 Mol) Natriumhydroxid in 120 ml Wasser tropft man unter Beibehaltung einer Reaktionstemperatur zwischen 0 und - 5°C langsam 12,5 g (0,079 Mol) Brom, versetzt anschließend mit 100 ml gekühltem Dioxan (14°C).

Die Lösung tropft man nun langsam zu einer gekühlten Lösung aus 0,0242 Mol des 2-Acetylindols so zu, daß die Reaktionstemperatur zwischen 10 und 15°C gehalten wird.

Man rührt weitere 4 Stunden bei 15 - 20°C, versetzt sodann mit einer Lösung aus 0,033 Mol Natriumsulfit in 40 ml Wasser. Das Reaktionsgemisch wird 30 Minuten am Rückflußkühler gekocht und nach Abkühlen auf etwa 60°C mit ca. 30 - 40 ml conc. HCl versetzt. Nach Kühlung im Eisbad filtriert man die Kristalle ab.

### Beispiel I/1):

5-Chlor-1,3-dimethylindol-2-carbonsäure, Fp. 244 - 247°C.

### Beispiel I/2):

5,7-Dichlor-1,3-dimethylindol-2-carbonsäure, Fp. 228 - 232°C.

### Beispiel I/3):

4,6-Dichlor-1,3-dimethylindol-2-carbonsäure, Fp. 212°C.

### Beispiel I/4):

5-Chlor-1-methyl-3-phenylindol-2-carbonsäure, Fp. 218 - 220°C.

### Beispiel I/5):

4,5,6-Trichlor-1,3-dimethylindol-2-carbonsäure, Fp. 232 - 237°C (Zersetzung)

### Beispiel I/6):

5-Benzoyl-1-methyl-1,3-dimethylindol-2-carbonsäure, Fp. 238 - 240°C.

### Sonstige Vorstufen

### Sonstige Vorstufen 1:

3-Isopropyl-5-methoxyindol-2-carbonsäureethylester
Man setzt 0,01 Mol 4-Methoxyphenylhydrazin Hydrochlorid mit 4-Methyl-2-ketovaleriansäureethylester in siedendem wasserfreiem Ethanol um (Kochdauer: 18 Stunden), dann wird das Lösungsmittel verdampft, sodann versetzt man mit Wasser, extrahiert mit Essigester, wäscht die organische Phase mit wäßriger Kochsalzlösung, trocknet und destilliert das Lösungsmittel ab. Umkristallisieren aus n-Heptan. Farblose Kristalle, Fp. 83 - 90°C.

### Sonstige Vorstufen 2: (Ullmann-Arylierung am Indol):

Eine Mischung aus 0,015 Mol Indol-2-carbonsäure, 50 ml wasserfreiem Dimethylformamid, 0,0165 Mol Brombenzol, 0,5 g Cu(II)oxid und 0,032 Mol KOH wird unter Rühren unter Argonatmosphäre 5 Stunden am Rückflußkühler gekocht. Man gießt die dunkle Suspension in Eiswasser, rührt 30 Minuten, filtriert durch eine Klärschicht oder eine Schicht Aktivkohle und behandelt das Filtrat nochmals mit Aktivkohle. Nach Filtration und Acidifizierung mit conc. HCl auf pH 1 - 2 wird die gewünschte Säure ausgefällt, filtriert, mehrmals mit Wasser gewaschen und getrocknet.

### Sonstige Vorstufen 2 a): 5-Methoxy-1-phenylindol-2-carbonsäure; Farblose Kristalle, Fp. 194 - 197°C

### Pharmakologische Daten:

### Inhibition des Na⁺/H⁺-Exchangers von Kaninchenerythrocyten

Weiße Neuseeland-Kaninchen (Ivanovas) erhielten eine Standard-Diät mit 2% Cholesterin für sechs Wochen, um den Na⁺/H⁺-Austausch zu aktivieren und so den Na⁺-Influx in die Erythrocyten via Na⁺/H⁺-Austausch flammenphotometrisch bestimmen zu können. Das Blut wurde den Ohrarterien entnommen und durch 25 IE Kalium-Heparin ungerinnbar gemacht. Ein Teil jeder Probe wurde zur Doppelbestimmung des Hämatokrits durch Zentrifugieren benutzt. Aliquots von jeweils 100 µl dienten zur Messung des Na⁺-Ausgangsgehalts der Erythrocyten.

Um den Amilorid-sensitiven Natrium-Influx zu bestimmen, wurden 100 µl jeder Blutprobe in jeweils 5 ml eines hyperosmolaren Salz-Sucrose-Mediums (mmol/l: 140 NaCl, 3 KCl, 150 Sucrose, 0,1 Ouabain, 20 Tris-hydroxymethylaminomethan) bei pH 7,4 und 37°C inkubiert. Die Erythrocyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na⁺ -Nettoinflux wurde aus der Differenz zwischen Natrium-Ausgangswerten und dem Natriumgehalt der Erythrocyten nach Inkubation errechnet. Der Amilorid-hemmbare Natrium-Influx ergab sich aus der Differenz des Natriumgehalts der Erythrocyten nach Inkubation mit und ohne Amilorid 3 x 10⁻⁴ mol/l. Auf diese Weise wurde auch bei den erfindungsgemäßen Verbindungen verfahren.

### Ergebnisse

| Inhibition des Na⁺/H⁺-Exchangers: | |
|---|---|
| Beispiel | IC₅₀ mol/l |
| 52 | 4 x 10⁻⁸ |
| 53 | 5 x 10⁻⁸ |
| 54 | 2 x 10⁻⁷ |
| 61 | 3 x 10⁻⁷ |
| 69 | 5 x 10⁻⁸ |
| 86 | 3 x 10⁻⁸ |
| 104 | 2 x 10⁻⁷ |
| 106 | 5 x 10⁻⁸ |

## Patentansprüche

1. Benzokondensierte 5-Ringheterocyclen der Formel 1 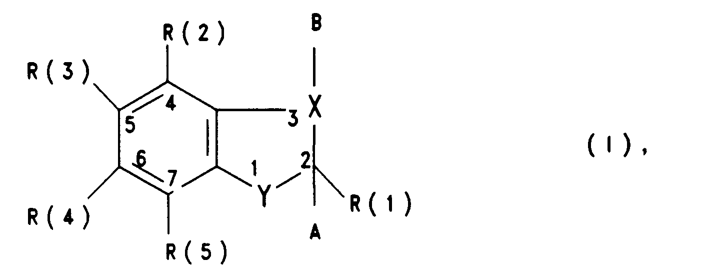 worin bedeuten:
X N, CR(6),
Y Sauerstoff, S, NR(7),
A, B gemeinsam eine Bindung
oder
beide Wasserstoff, sofern gleichzeitig X gleich CR(6) und Y gleich NR(7) sind,
einer der Substituenten R(1) bis R(6) eine -CO-N = C(NH₂)₂-Gruppe, die jeweils anderen Substituenten R(1) bis R(6)
Wasserstoff, F, Cl, Br, I, (C₁-C₆)-Alkyl,
bis zu zwei der anderen Substituenten R(1) bis R(6)
CN, NO₂, N₃, (C₁-C₄)-Alkyloxy, CF_{3,}
bis zu einem der anderen Substituenten
R(8)-CₙH₂ₙ-Z-,
n Null bis 10,
wobei die Alkylenkette -CₙH₂ₙ- geradkettig oder verzweigt ist und wobei ein C-Atom durch ein Sauerstoff- oder S-Atom oder durch ein N-Atom ersetzt sein kann,
R(8) Wasserstoff, (C₂-C₆)-Alkenyl, (C₃-C₁₀)-Cycloalkyl,
das unsubstituiert oder durch 1 bis 4 Methylgruppen oder eine OH-Gruppe substituiert ist, oder eine Ethylengruppe -CH = CH- enthalten kann, und worin eine Methylengruppe durch ein Sauerstoff- oder S-Atom oder durch ein N-Atom ersetzt sein kann,
Phenyl,
unsubstituiert oder substituiert durch 1 bis 3 Substituenten ausgewählt aus der Gruppe bestehend aus F, Cl, Br, I, CF₃, CH₃-S(O)ₛ-,
s Null, 1, 2,
R(9)-W_{y}-,
R(9) H, Methyl, Ethyl,
W gleich Sauerstoff, NR(10),
R(10) H, Methyl,
y Null, 1,
CₘF₂ₘ₊₁,
m 1 bis 3,
1- oder 2-Naphthyl, Pyridyl, Chinolyl, Isochinolyl,
Z -CO-, -CH₂-, -[CR(11)(OH)]_{q}-
q 1, 2, 3,
R(11) H, Methyl,
Sauerstoff, -NR(12)-,
R(12) H, Methyl,
-S(O)ₛ-,
s Null, 1, 2,
-SO₂-NR(13)-,
R(13) H, (C₁-C₄)-Alkyl,
R(7) Wasserstoff, (C₁-C₁₀)-Alkyl, (C₂-C₁₀)-Alkenyl, R(8)-CₙH₂ₙ-,
sowie deren pharmazeutisch verträgliche Salze.

2. Verbindungen der Formel I nach Anspruch 1, dadurch gekennzeichnet, daß darin bedeuten:
X CR(6), N,
Y NR(7),
A, B gemeinsam eine Bindung
oder
beide Wasserstoff, sofern gleichzeitig X gleich CR(6) und Y gleich NR(7) sind,
und die Reste R(1) bis R(7) die angegebene Bedeutung besitzen.

3. Verbindungen der Formel I nach Anspruch 1, dadurch gekennzeichnet, daß darin bedeuten:
X CR(6), N, Y NR(7),
A, B gemeinsam eine Bindung
oder
beide Wasserstoff, sofern gleichzeitig X gleich CR(6) und Y gleich NR(7) sind,
einer der Substituenten R(1) bis R(6) eine -CO-N = C(NH₂)₂-Gruppe, und die jeweils anderen Substituenten R(1) bis R(6)
Wasserstoff, F, Cl, Br, I, (C₁-C₆)-Alkyl,
bis zu zwei der anderen Substituenten R(1) bis R(6)
CF₃, (C₁-C₄)-Alkyloxy,
bis zu einem der anderen Substituenten R(1) bis R(6) CN, NO₂, N₃, R(8)-CₙH₂ₙ-Z-,
n Null bis 4, wobei die Alkylenkette -CₙH₂ₙ- geradkettig oder verzweigt sein kann und ein C-Atom durch ein Sauerstoff oder S-Atom oder durch ein N-Atom ersetzt sein kann,
R(8) Wasserstoff, (C₃-C₆)-Alkenyl, (C₅-C₈)-Cycloalkyl,
das unsubstituiert ist oder durch 1 - 2 Methylgruppen oder eine OH-Gruppe substituiert, und worin eine Methylengruppe durch ein Sauerstoff- oder S-Atom oder durch ein N-Atom ersetzt sein kann,
Phenyl,
das unsubstituiert ist oder substituiert mit 1 bis 3 Substituenten ausgewählt aus der Gruppe bestehend aus F, Cl, Br, I, CF₃, CH₃-S(O)ₛ-
s Null, 1, 2,
R(9)-W_{y}-
R(9) H, Methyl, Ethyl,
W Sauerstoff, NR(10),
R(10) H, Methyl,
y Null, 1,
CₘF₂ₘ₊₁,
m 1 bis 3,
Pyridyl, Chinolyl, Isochinolyl
Z -CO-, -CH₂-, Sauerstoff, -NR(12)-,
R(12) H, Methyl,
-S(O)ₛ-,
s Null, 1, 2,
-SO₂-NR(13)-,
R(13) H, (C₁-C₄)-Alkyl,
R(7) Wasserstoff, (C₁-C₆)-Alkyl, (C₂-C₄)-Alkenyl, R(8)-CₙH₂ₙ-.

4. Verbindungen der Formel I nach Anspruch 1, dadurch gekennzeichnet, daß darin bedeuten:
X CR(6),
Y NR(7),
A, B gemeinsam eine Bindung
oder
beide Wasserstoff, sofern gleichzeitig X gleich CR(6) und Y gleich NR(7) sind,
und R(1) -CO-N= C(NH₂)₂
und die jeweils anderen Substituenten R(2) bis R(6)
Wasserstoff, F, Cl, Br,
bis zu zwei der Substituenten R(2) bis R(6)
CF₃, (C₁-C₂)-Alkyloxy,
bis zu einem der Substituenten R(2) bis R(6)
R(8)-CₙH₂ₙ-Z-,
n Null, 1, 2,
wobei die Alkylenkette -CₙH₂ₙ- geradkettig oder verzweigt ist und wobei ein C-Atom durch ein Sauerstoff- oder S-Atom oder durch ein N-Atom ersetzt sein kann,
R(8) Wasserstoff, Phenyl,
das unsubstituiert ist oder substituiert mit 1 bis 3 Substituenten ausgewählt aus der Gruppe bestehend aus F, Cl, CF₃, CH₃-S(O)ₛ-,
s Null, 2,
R(9)-W_{y}-,
R(9) H, Methyl,
W Sauerstoff,
y Null, 1,
Pyridyl, Chinolyl, Isochinolyl,
Z -CO-, -CH₂-, -S(O)ₛ-, Sauerstoff,
s Null, 1, 2,
und
R(7) (C₁-C₆)-Alkyl, (C₃-C₄)-Alkenyl, R(8)-CₙH₂ₙ-,

5. Verbindung der Formel I nach Anspruch 1, dadurch gekennzeichnet, daß sie ausgewählt ist aus der Gruppe bestehend aus
5-Chlor-1-methyl-2-indolylcarbonyl-guanidin-hydrochlorid,
5-Chlor-1-ethyl-2-indolylcarbonyl-guanidin-hydrochlorid,
3-Chlor-1-methyl-2-indolylcarbonyl-guanidin-hydrochlorid,
3,5-Dichlor-1-methyl-2-indolylcarbonyl-guanidin-hydrochlorid,
5-Fluor-1-methyl-2-indolylcarbonyl-guanidin-hydrochlorid,
3-Chlor-5-fluor-1-methyl-2-indolylcarbonyl-guanidin-hydrochlorid,
4,6-Dichlor-1,3-dimethyl-2-indolylcarbonyl-guanidin-hydrochlorid,
2-Phenoxy-1-phenylindol-3-carbonsäureguanidid Methansulfonsäuresalz,
2-Chlor-1-phenylindol-3-carbonsäureguanidid Methansulfonsäuresalz,
1-Methylindolin-2-carbonsäure-guanidid-hydrochlorid,
5-Fluor-1-methylindolin-2-carbonsäure-guanidid-hydrochlorid.

6. Verfahren zur Herstellung einer Verbindung der Formel I nach Anspruch 1, dadurch gekennzeichnet, daß man
eine Verbindung der Formel II 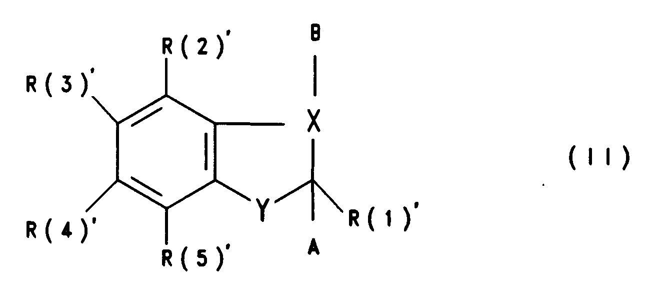 worin einer der Substituenten R(1)' bis R(5)' eine -CO-L -Gruppe bedeutet und L für eine leicht nucleophil substituierbare leaving group steht, und die jeweils anderen Substituenten R(1)' bis R(5)' die angegebene Bedeutung besitzen,
mit Guanidin unter Bildung der in Formel I angegebenen Acylguanidingruppe,
-CO-N = C(NH₂)₂,
umsetzt,
und daß man gegebenenfalls in das pharmazeutisch verträgliche Salz überführt.

7. Verwendung einer Verbindung I nach Anspruch 1 zur Herstellung eines Medikaments zur Behandlung von Arrhythmien.

8. Methode zum Behandeln von Arrhythmien, dadurch gekennzeichnet, daß man eine wirksame Menge einer Verbindung I nach Anspruch 1 mit den üblichen Zusatzstoffen versetzt und in einer geeigneten Darreichungsform verabreicht.

9. Verwendung einer Verbindung I nach Anspruch 1 zur Herstellung eines Medikaments zur Behandlung oder Prophylaxe des Herzinfarktes.

10. Verwendung einer Verbindung I nach Anspruch 1 zur Herstellung eines Medikaments zur Behandlung oder Prophylaxe der Angina Pectoris.

11. Verwendung einer Verbindung I nach Anspruch 1 zur Herstellung eines Medikaments zur Behandlung oder Prophylaxe von ischämischen Zuständen des Herzens.

12. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

13. Verwendung einer Verbindung I nach Anspruch 1 zur Herstellung eines Medikaments zur Behandlung oder Prophylaxe von ischämischen Zuständen peripherer Organe und Gliedmaßen.

14. Verwendung einer Verbindung I nach Anspruch 1 zur Herstellung eines Medikaments zur Behandlung von Schockzuständen.

15. Verwendung einer Verbindung I nach Anspruch 1 zur Herstellung eines Medikaments zum Einsatz bei chirurgischen Operationen und Organtransplantationen.

16. Verwendung einer Verbindung I nach Anspruch 1 zur Herstellung eines Medikaments zur Konservierung und Lagerung von Transplantaten für chirurgische Maßnahmen.

17. Verwendung einer Verbindung I nach Anspruch I zur Herstellung eines Medikaments zur Behandlung von Krankheiten, bei denen die Zellproliferation eine primäre oder sekundäre Ursache darstellt, und somit ihre Verwendung als Antiatherosklerotika, Mittel gegen Diabetische Spätkomplikationen, Krebserkrankungen, fibrotische Erkrankungen wie Lungenfibrose, Leberfibrose oder Nierenfibrose, Prostatahyperplasie.

18. Verwendung einer Verbindung I nach Anspruch I zur Herstellung eines wissenschaftliches Tools zur Inhibition des Na⁺/H⁺-Exchangers, zur Diagnose der Hypertonie und proliferativer Erkrankungen.

19. Heilmittel, enthaltend eine wirksame Menge einer Verbindung I nach Anspruch 1.
